# EUROPEAN PATENT SPECIFICATION

(11) **EP 3 873 366 B1**
(45) Date of publication and mention of the grant of the patent: **24.09.2025**
(21) Application number: 19795355.7
(22) Date of filing: 24.10.2019
(51) Int. Cl.: A61B 18/14, A61B 34/00, A61B 34/30

(54) **DISTAL CLOSURE MECHANISM FOR SURGICAL INSTRUMENTS**
DISTALER VERSCHLUSSMECHANISMUS FÜR CHIRURGISCHE INSTRUMENTE
MÉCANISME DE FERMETURE DISTAL POUR INSTRUMENTS CHIRURGICAUX

(30) Priority: 02.11.2018 US 201816179208
(43) Date of publication of application: 08.09.2021
(73) Proprietor: Cilag GmbH International, 6300 Zug (CH)
(72) Inventor: DICKERSON, Benjamin D., Cincinnati, Ohio 45242 (US); DAVISON, Mark A., Cincinnati, Ohio 45242 (US); BIRRI, Christopher W., Cincinnati, Ohio 45242 (US)
(74) Representative: Carpmaels & Ransford LLP
(86) International application number: PCT/IB2019/059148
(87) International publication number: WO 2020/089749

(56) References cited:
- US-A1- 2017 252 096
- US-A1- 2018 311 471
- US-B1- 8 333 780

## Description

### BACKGROUND

Minimally invasive surgical (MIS) instruments are often preferred over traditional open surgical devices due to reduced post-operative recovery time and minimal scarring. Laparoscopic surgery is one type of MIS procedure in which one or more small incisions are formed in the abdomen of a patient and a trocar is inserted through the incision to form a pathway that provides access to the abdominal cavity. Through the trocar, a variety of instruments and surgical tools can be introduced into the abdominal cavity. The instruments and tools introduced into the abdominal cavity via the trocar can be used to engage and/or treat tissue in a number of ways to achieve a diagnostic or therapeutic effect.

Various robotic systems have recently been developed to assist in MIS procedures. Robotic systems can allow for more instinctive hand movements by maintaining natural eye-hand axis. Robotic systems can also allow for more degrees of freedom in movement by including an articulable "wrist" joint that creates a more natural hand-like articulation. In such systems, an end effector positioned at the distal end of the instrument can be articulated (moved) using a cable driven motion system having one or more drive cables that extend through the wrist joint. A user (e.g., a surgeon) is able to remotely operate the end effector by grasping and manipulating in space one or more controllers that communicate with a tool driver coupled to the surgical instrument. User inputs are processed by a computer system incorporated into the robotic surgical system, and the tool driver responds by actuating the cable driven motion system. Moving the drive cables articulates the end effector to desired angular positions and configurations.

Some end effectors have opposing jaws that are opened and closed during operation to undertake various surgical treatments. It is desirable to incorporate compact and efficient mechanisms that open and close the jaws.

US2018311471A1 relates to a sliding distal component assembly for use in medical catheters, bioptomes and other medical devices. The sliding distal component assembly includes a tubular shaft having a lumen, the tubular shaft coupled to an actuation mechanism moveable between a first position and a second position. A tip support is coupled to the tubular shaft and defines a tip support cavity therewithin. The sliding component assembly includes a pulley system having at least one pulley, a sliding distal component and at least one pull wire coupled to the pulley and the sliding distal component with at least a portion of said pulley system being housed within said tip support cavity. When the actuation mechanism is in the first position the sliding distal component is configured to translate proximally and when the actuation mechanism is in the second position the sliding distal component is configured to translate distally.

US2017252096A1 relates to various exemplary systems, devices, and methods for robotic bi-polar instruments. In general, a surgical tool can include an elongate shaft, an end effector, a wrist that couples the end effector to the shaft at a distal end of the shaft, and a tool housing coupled to a proximal end of the shaft that is configured to control the operation various features associated with the end effector and to operatively couple to a robotic surgical system.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure.
FIG. 1 is a block diagram of an example robotic surgical system that may incorporate some or all of the principles of the present disclosure.
FIG. 2 is an isometric side view of an example surgical tool that may incorporate some or all of the principles of the present disclosure.
FIG. 3 illustrates potential degrees of freedom in which the wrist of the surgical tool of FIG. 2 may be able to articulate (pivot).
FIG. 4 is an enlarged isometric view of the distal end of the surgical tool of FIG. 2.
FIGS. 5A and 5B are isometric and side views, respectively, of the end effector of FIG. 4 with the jaws closed, according to one or more embodiments.
FIGS. 6A and 6B are isometric and side views, respectively, of the end effector of FIG. 4 with the jaws open, according to one or more embodiments.

### DETAILED DESCRIPTION

The present invention relates to robotic surgical systems and, more particularly, to cable-driven end effectors with an improved and compact closure/opening mechanism.

In particular, the present invention provides an end effector according to claim 1, in which a first jaw is rotatably coupled to a second jaw at a jaw axle, a central pulley is rotatably mounted to the jaw axle, and a pivot link is rotatably coupled to the first jaw at a pivot axle. A single one of the first and second jaws is configured to pivot to articulate the end effector between open and closed positions. A jaw cable is looped around the central pulley and operatively coupled to the pivot link such that linear movement of the jaw cable correspondingly causes the first jaw to rotate relative to the second jaw on the jaw axle and between the open and closed positions. The jaw cable may include two proximally extending ends that terminate at separate actuation mechanisms (e.g., capstans) within a drive housing. Accordingly, the drive cable may work on a "pull-pull" strategy to open and close the jaws. Further embodiments of the invention are described in the dependent claims.

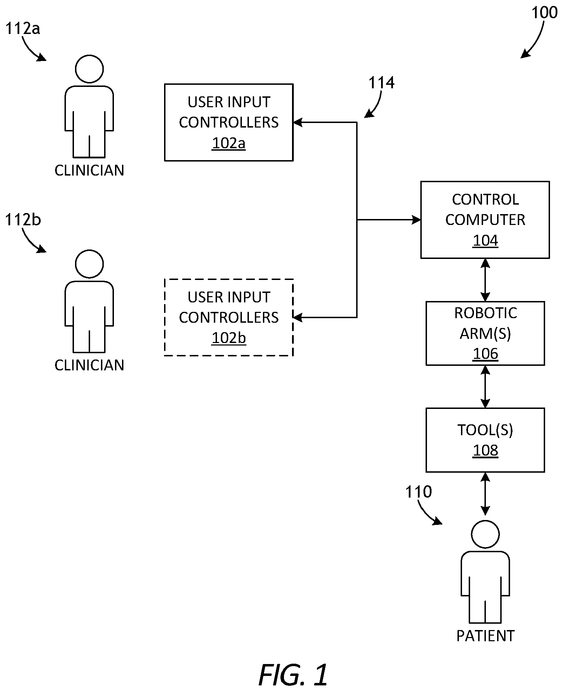

FIG. 1 is a block diagram of an example robotic surgical system 100 that may incorporate some or all of the principles of the present disclosure. As illustrated, the system 100 can include at least one set of user input controllers 102a and at least one control computer 104. The control computer 104 may be mechanically and/or electrically coupled to a robotic manipulator and, more particularly, to one or more robotic arms 106 (alternately referred to as "tool drivers"). In some embodiments, the robotic manipulator may be included in or otherwise mounted to an arm cart capable of making the system portable. Each robotic arm 106 may include and otherwise provide a location for mounting one or more surgical instruments or tools 108 for performing various surgical tasks on a patient 110. Operation of the robotic arms 106 and associated tools 108 may be directed by a clinician 112a (e.g., a surgeon) from the user input controller 102a.

In some embodiments, a second set of user input controllers 102b (shown in dashed lines) may be operated by a second clinician 112b to direct operation of the robotic arms 106 and tools 108 in conjunction with the first clinician 112a. In such embodiments, for example, each clinician 112a,b may control different robotic arms 106 or, in some cases, complete control of the robotic arms 106 may be passed between the clinicians 112a,b. In some embodiments, additional robotic manipulators (not shown) having additional robotic arms (not shown) may be utilized during surgery on the patient 110, and these additional robotic arms may be controlled by one or more of the user input controllers 102a,b.

The control computer 104 and the user input controllers 102a,b may be in communication with one another via a communications link 114, which may be any type of wired or wireless telecommunications means configured to carry a variety of communication signals (e.g., electrical, optical, infrared, etc.) according to any communications protocol. In some applications, for example, there is a tower with ancillary equipment and processing cores designed to drive the robotic arms 106.

The user input controllers 102a,b generally include one or more physical controllers that can be grasped by the clinicians 112a,b and manipulated in space while the surgeon views the procedure via a stereo display. The physical controllers generally comprise manual input devices movable in multiple degrees of freedom, and which often include an actuatable handle for actuating the surgical tool(s) 108, for example, for opening and closing opposing jaws, applying an electrical potential (current) to an electrode, or the like. The control computer 104 can also include an optional feedback meter viewable by the clinicians 112a,b via a display to provide a visual indication of various surgical instrument metrics, such as the amount of force being applied to the surgical instrument (i.e., a cutting instrument or dynamic clamping member).

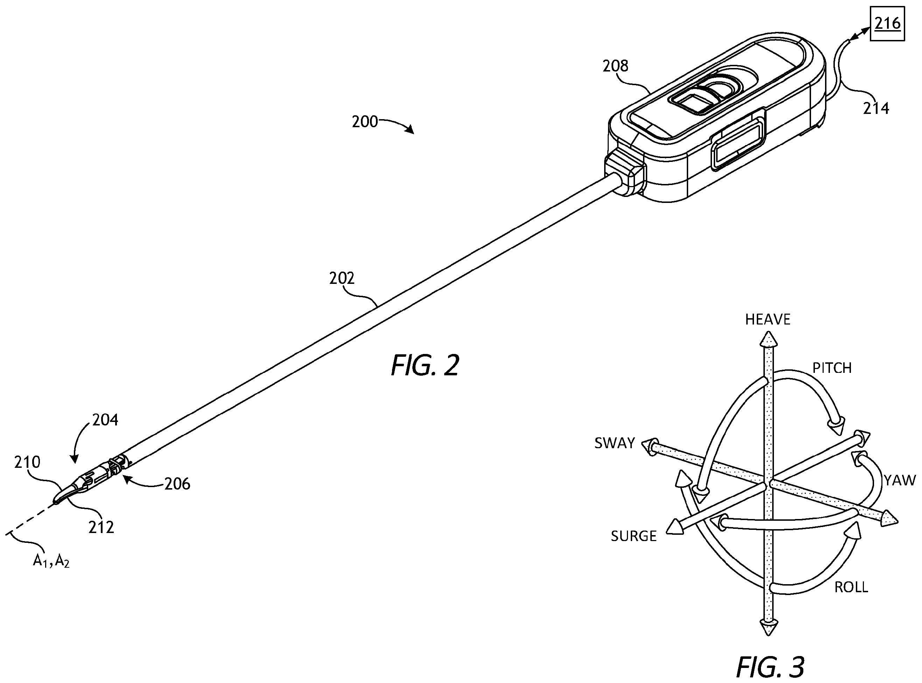

FIG. 2 is an isometric side view of an example surgical tool 200 that may incorporate some or all of the principles of the present disclosure. The surgical tool 200 may be the same as or similar to the surgical tool(s) 108 of FIG. 1 and, therefore, may be used in conjunction with a robotic surgical system, such as the robotic surgical system 100 of FIG. 1. Accordingly, the surgical tool 200 may be designed to be releasably coupled to a tool driver included in the robotic surgical system 100. In other embodiments, however, aspects of the surgical tool 200 may be adapted for use in a manual or hand-operated manner, without departing from the scope of the disclosure.

As illustrated, the surgical tool 200 includes an elongated shaft 202, an end effector 204, a wrist 206 (alternately referred to as a "wrist joint" or an "articulable wrist joint") that couples the end effector 204 to the distal end of the shaft 202, and a drive housing 208 coupled to the proximal end of the shaft 202. In applications where the surgical tool is used in conjunction with a robotic surgical system (e.g., the robotic surgical system 100 of FIG. 1), the drive housing 208 can include coupling features that releasably couple the surgical tool 200 to the robotic surgical system.

The terms "proximal" and "distal" are defined herein relative to a robotic surgical system having an interface configured to mechanically and electrically couple the surgical tool 200 (e.g., the housing 208) to a robotic manipulator. The term "proximal" refers to the position of an element closer to the robotic manipulator and the term "distal" refers to the position of an element closer to the end effector 204 and thus further away from the robotic manipulator. Alternatively, in manual or hand-operated applications, the terms "proximal" and "distal" are defined herein relative to a user, such as a surgeon or clinician. The term "proximal" refers to the position of an element closer to the user and the term "distal" refers to the position of an element closer to the end effector 204 and thus further away from the user. Moreover, the use of directional terms such as above, below, upper, lower, upward, downward, left, right, and the like are used in relation to the illustrative embodiments as they are depicted in the figures, the upward or upper direction being toward the top of the corresponding figure and the downward or lower direction being toward the bottom of the corresponding figure.

During use of the surgical tool 200, the end effector 204 is configured to move (pivot) relative to the shaft 202 at the wrist 206 to position the end effector 204 at desired orientations and locations relative to a surgical site. To accomplish this, the housing 208 includes (contains) various drive inputs and mechanisms (e.g., gears, actuators, etc.) designed to control operation of various features associated with the end effector 204 (e.g., clamping, firing, rotation, articulation, cutting, etc.). In at least some embodiments, the shaft 202, and hence the end effector 204 coupled thereto, is configured to rotate about a longitudinal axis A₁ of the shaft 202. In such embodiments, at least one of the drive inputs included in the housing 208 is configured to control rotational movement of the shaft 202 about the longitudinal axis A₁.

The surgical tool 200 can have any of a variety of configurations capable of performing at least one surgical function. For example, the surgical tool 200 may include, but is not limited to, forceps, a grasper, a needle driver, scissors, an electro cautery tool, a stapler, a clip applier, a hook, a spatula, a suction tool, an irrigation tool, an imaging device (e.g., an endoscope or ultrasonic probe), or any combination thereof. In some embodiments, the surgical tool 200 may be configured to apply energy to tissue, such as radio frequency (RF) energy.

The shaft 202 is an elongate member extending distally from the housing 208 and has at least one lumen extending therethrough along its axial length. In some embodiments, the shaft 202 may be fixed to the housing 208, but could alternatively be rotatably mounted to the housing 208 to allow the shaft 202 to rotate about the longitudinal axis A₁. In yet other embodiments, the shaft 202 may be releasably coupled to the housing 208, which may allow a single housing 208 to be adaptable to various shafts having different end effectors.

The end effector 204 can have a variety of sizes, shapes, and configurations. In the illustrated embodiment, the end effector 204 comprises a tissue grasper and vessel sealer that include opposing jaws 210, 212 configured to move (articulate) between open and closed positions. As will be appreciated, however, the opposing jaws 210, 212 may alternatively form part of other types of end effectors such as, but not limited to, a surgical scissors, a clip applier, a needle driver, a babcock including a pair of opposed grasping jaws, bipolar jaws (e.g., bipolar Maryland grasper, forceps, a fenestrated grasper, etc.), etc. A single one of the jaws 210, 212 is configured to pivot to articulate the end effector 204 between the open and closed positions.

FIG. 3 illustrates the potential degrees of freedom in which the wrist 206 may be able to articulate (pivot). The wrist 206 can have any of a variety of configurations. In general, the wrist 206 comprises a joint configured to allow pivoting movement of the end effector 204 relative to the shaft 202. The degrees of freedom of the wrist 206 are represented by three translational variables (i.e., surge, heave, and sway), and by three rotational variables (i.e., Euler angles or roll, pitch, and yaw). The translational and rotational variables describe the position and orientation of a component of a surgical system (e.g., the end effector 204) with respect to a given reference Cartesian frame. As depicted in FIG. 3, "surge" refers to forward and backward translational movement, "heave" refers to translational movement up and down, and "sway" refers to translational movement left and right. With regard to the rotational terms, "roll" refers to tilting side to side, "pitch" refers to tilting forward and backward, and "yaw" refers to turning left and right.

The pivoting motion can include pitch movement about a first axis of the wrist 206 (e.g., X-axis), yaw movement about a second axis of the wrist 206 (e.g., Y-axis), and combinations thereof to allow for 360° rotational movement of the end effector 204 about the wrist 206. In other applications, the pivoting motion can be limited to movement in a single plane, e.g., only pitch movement about the first axis of the wrist 206 or only yaw movement about the second axis of the wrist 206, such that the end effector 204 moves only in a single plane.

Referring again to FIG. 2, the surgical tool 200 may also include a plurality of drive cables (obscured in FIG. 2) that form part of a cable driven motion system configured to facilitate movement and articulation of the end effector 204 relative to the shaft 202. Moving (actuating) at least some of the drive cables moves the end effector 204 between an unarticulated position and an articulated position. The end effector 204 is depicted in FIG. 2 in the unarticulated position where a longitudinal axis A₂ of the end effector 204 is substantially aligned with the longitudinal axis A₁ of the shaft 202, such that the end effector 204 is at a substantially zero angle relative to the shaft 202. Due to factors such as manufacturing tolerance and precision of measurement devices, the end effector 204 may not be at a precise zero angle relative to the shaft 202 in the unarticulated position, but nevertheless be considered "substantially aligned" thereto. In the articulated position, the longitudinal axes A₁, A₂ would be angularly offset from each other such that the end effector 204 is at a non-zero angle relative to the shaft 202.

In some embodiments, the surgical tool 200 may be supplied with electrical power (current) via a power cable 214 coupled to the housing 208. In other embodiments, the power cable 214 may be omitted and electrical power may be supplied to the surgical tool 200 via an internal power source, such as one or more batteries or fuel cells. In such embodiments, the surgical tool 200 may alternatively be characterized and otherwise referred to herein as an "electrosurgical instrument" capable of providing electrical energy to the end effector 204.

The power cable 214 may place the surgical tool 200 in communication with a generator 216 that supplies energy, such as electrical energy (e.g., radio frequency energy), ultrasonic energy, microwave energy, heat energy, or any combination thereof, to the surgical tool 200 and, more particularly, to the end effector 204. Accordingly, the generator 216 may comprise a radio frequency (RF) source, an ultrasonic source, a direct current source, and/or any other suitable type of electrical energy source that may be activated independently or simultaneously.

In applications where the surgical tool 200 is configured for bipolar operation, the power cable 214 will include a supply conductor and a return conductor. Current can be supplied from the generator 216 to an active (or source) electrode located at the end effector 204 via the supply conductor, and current can flow back to the generator 216 via a return electrode located at the end effector 204 via the return conductor. In the case of a bipolar grasper with opposing jaws, for example, the jaws serve as the electrodes where the proximal end of the jaws are isolated from one another and the inner surface of the jaws (i.e., the area of the jaws that grasp tissue) apply the current in a controlled path through the tissue. In applications where the surgical tool 200 is configured for monopolar operation, the generator 216 transmits current through a supply conductor to an active electrode located at the end effector 204, and current is returned (dissipated) through a return electrode (e.g., a grounding pad) separately coupled to a patient's body.

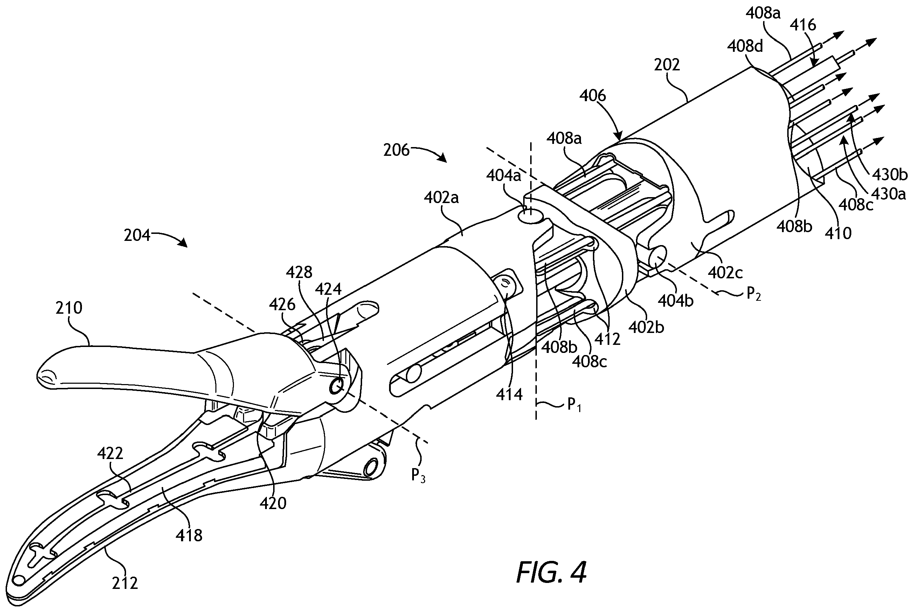

FIG. 4 is an enlarged isometric view of the distal end of the surgical tool 200 of FIG. 2. More specifically, FIG. 4 depicts enlarged views of the end effector 204 and the wrist 206, with the jaws 210, 212 of the end effector 204 in the open position. The wrist 206 operatively couples the end effector 204 to the shaft 202. In some embodiments, however, a shaft adapter may be directly coupled to the wrist 206 and otherwise interpose the shaft 202 and the wrist 206. Accordingly, the wrist 206 may be operatively coupled to the shaft 202 either through a direct coupling engagement where the wrist 206 is directly coupled to the distal end of the shaft 202, or an indirect coupling engagement where a shaft adapter interposes the wrist 206 and the distal end of the shaft 202. As used herein, the term "operatively couple" refers to a direct or indirect coupling engagement.

To operatively couple the end effector 204 to the shaft 202, the wrist 206 includes a first or "distal" linkage 402a, a second or "intermediate" linkage 402b, and a third or "proximal" linkage 402c. The linkages 402a-c are configured to facilitate articulation of the end effector 204 relative to the elongate shaft 202, e.g., angle the end effector 204 relative to the longitudinal axis A₁ (FIG. 2) of the shaft 202. In the illustrated embodiment, articulation via the linkages 402a-c may be limited to pitch only, yaw only, or a combination thereof. As illustrated, the distal linkage 402a may be coupled to the end effector 204 and, more particularly, to the lower jaw 212 (or an extension of the lower jaw 212). The distal linkage 402a may also be rotatably coupled to the intermediate linkage 402b at a first axle 404a, and the intermediate linkage 402b may be rotatably coupled to the proximal linkage 402c at a second axle 404b. The proximal linkage 402c may then be coupled to a distal end 406 of the shaft 202 (or alternatively a shaft adapter).

The wrist 206 provides a first pivot axis P₁ that extends through the first axle 404a and a second pivot axis P₂ that extends through the second axle 404b. The first pivot axis P₁ is substantially perpendicular (orthogonal) to the longitudinal axis A₂ (FIG. 2) of the end effector 204, and the second pivot axis P₂ is substantially perpendicular (orthogonal) to both the longitudinal axis A₂ and the first pivot axis P₁. Movement about the first pivot axis P₁ provides "yaw" articulation of the end effector 204, and movement about the second pivot axis P₂ provides "pitch" articulation of the end effector 204. Alternatively, the first pivot axis P₁ could be configured to provide "pitch" articulation and the second pivot axis P₂ could be configured to provide "yaw" articulation.

A plurality of drive cables, shown as drive cables 408a, 408b, 408c, and 408d, extend longitudinally within a lumen 410 defined by the shaft 202 (and/or a shaft adaptor) and pass through the wrist 206 to be operatively coupled to the end effector 204. The lumen 410 can be a single lumen, as illustrated, or can alternatively comprise a plurality of independent lumens that each receive one or more of the drive cables 408a-d.

The drive cables 408a-d form part of the cable driven motion system briefly described above, and may be referred to and otherwise characterized as cables, bands, lines, cords, wires, ropes, strings, twisted strings, elongate members, etc. The drive cables 408a-d can be made from a variety of materials including, but not limited to, metal (e.g., tungsten, stainless steel, etc.), a polymer (e.g., ultra-high molecular weight polyethylene), a synthetic fiber (e.g., KEVLAR^{®}, VECTRAN^{®}, etc.), or any combination thereof. While four drive cables 408a-d are depicted in FIG. 4, more or less than four drive cables 408a-d may be included, without departing from the scope of the disclosure.

The drive cables 408a-d extend proximally from the end effector 204 to the drive housing 208 (FIG. 2) where they are operatively coupled to various actuation mechanisms (e.g., capstans) or devices housed therein to facilitate longitudinal movement (translation) of the drive cables 408a-d within the lumen 410. Selective actuation of all or a portion of the drive cables 408a-d causes the end effector 204 to articulate (pivot) relative to the shaft 202. More specifically, selective actuation causes a corresponding drive cable 408a-d to translate longitudinally within the lumen 410 and thereby cause pivoting movement of the end effector 204. Moving the drive cables 408a-d can be accomplished in a variety of ways, such as by triggering an associated actuator or mechanism (e.g., a capstan) operatively coupled to or housed within the drive housing 208 (FIG. 2). Moving a given drive cable 408a-d constitutes applying tension (i.e., pull force) to the given drive cable 408a-d in a proximal direction, which causes the given drive cable 408a-d to translate and thereby cause the end effector 204 to move (articulate) relative to the shaft 202. As will be appreciated, applying tension to and moving one drive cable 408a-d may result in the slackening of a drive cable 402a-d angularly (or diagonally) opposite to the moving drive cable 402a-d.

The drive cables 408a-d each extend longitudinally through the first, second, and third linkages 402a-c. In some embodiments, each linkage 402a-c may define four, equidistantly-spaced apertures 412 (only two labeled) configured to guide the drive cables 408a-d through the wrist 206. The apertures 412 of each linkage 402a-c may coaxially align when the end effector 204 is in the unarticulated position. The apertures 412 may provide rounded edges and sufficiently large radii to help reduce friction between the drive cables 408a-d and the linkages 402a-c and/or help prevent the drive cables 408a-d from twisting or moving radially inward or outward during articulation.

In some embodiments, the distal end of each drive cable 408a-d may terminate at the first linkage 402a, thus operatively coupling each drive cable 408a-d to the end effector 204 and, more particularly, to the lower jaw 212. The distal end of each drive cable 408a-d may be enlarged to facilitate fixed attachment thereof to the end effector 204. In some embodiments, as illustrated, the distal end of each drive cable 408a-d may include a ball crimp 412 (only one shown). In other embodiments, the distal end of each drive cable 408a-d may include a weld, an adhesive attachment, a press fit, or any combination of the foregoing.

In one or more embodiments, an electrical conductor 416 may supply electrical energy to the end effector 204 and, more particularly, to an electrode 418 included in the end effector 204. The electrical conductor 416 extends longitudinally within the lumen 410, through the wrist 206, and terminates at the electrode 418. In the illustrated embodiment, the electrode 418 is mounted to or otherwise forms part of the lower jaw 212. In other embodiments, however, the electrode 418 may form part of the upper jaw 210, or may alternatively be coupled to or form part of both jaws 210, 212. In some embodiments, the electrical conductor 416 and the power cable 214 (FIG. 2) may comprise the same structure. In other embodiments, however, the electrical conductor 416 may be electrically coupled to the power cable 214, such as at the drive housing 208 (FIG. 2). In yet other embodiments, the electrical conductor 416 may extend to the drive housing 208 where it is electrically coupled to an internal power source, such as batteries or fuel cells.

In some embodiments, the electrical conductor 416 may comprise a wire. In other embodiments, however, the electrical conductor 416 may comprise a rigid or semi-rigid shaft, rod, or strip (ribbon) made of a conductive material. In some embodiments, the electrical conductor 416 may be partially covered with an insulative covering (overmold) made of a non-conductive material. The insulative covering, for example, may comprise a plastic applied to the electrical conductor 416 via heat shrinking, but could alternatively be any other non-conductive material.

The end effector 204 may be configured for monopolar or bipolar operation. In at least one embodiment, the electrical energy conducted through the electrical conductor 416 may comprise radio frequency ("RF") energy exhibiting a frequency between about 100 kHz and 1 MHz. In a process known as Joule heating (resistive or Ohmic heating) the RF energy is transformed into heat within the target tissue due the tissue's intrinsic electrical impedance, thereby increasing the temperature of target tissue. Accordingly, heating of the target tissue is used to achieve various tissue effects such as cauterization and/or coagulation and thus may be particularly useful for sealing blood vessels or diffusing bleeding during a surgical procedure.

In the illustrated embodiment, the end effector 204 comprises a vessel sealer that includes a cutting element 420 (mostly occluded) configured to traverse a groove or slot 422 defined longitudinally in one or both of the upper and lower jaws 210, 212. In example operation, the jaws 210, 212 may be actuated to close and grasp onto tissue, following which the cutting element 420 may be advanced distally along the slot(s) 422 to cut the grasped tissue. Alternatively, the cutting element 420 may be deployed after the application of electrical energy to transect coagulated tissue.

The jaws 210, 212 are moved between the closed and open positions by pivoting the upper jaw 210 relative to the lower jaw 212. In the illustrated embodiment, the upper jaw 210 is rotatably coupled (mounted) to the lower jaw 212 at a jaw axle 424. A third pivot axis P₃ extends through the jaw axle 424 and is generally perpendicular (orthogonal) to the first pivot axis P₁ and parallel to the second pivot axis P₂. A central pulley 426 (partially visible) is mounted to the jaw axle 424 and receives a jaw cable 428 that may be actuated to selectively open and close the jaws 210, 212.

Similar to the drive cables 408a-d, the jaw cable 428 extends longitudinally within the lumen 410 and passes through the wrist 206. Moreover, the jaw cable 428 may form part of the cable driven motion system described herein and, therefore, may extend proximally from the end effector 204 to the drive housing 208 (FIG. 2). In some embodiments, the jaw cable 428 comprises two lines or wires connected at or near the central pulley 426 and extending proximally to the drive housing 208. In other embodiments, however, the jaw cable 428 may comprise a single line or wire looped around the central pulley 426 and opposing first and second ends 430a and 430b of the jaw cable 428 extend proximally to the drive housing 208.

In some embodiments, the ends 430a,b of the jaw cable 428 may be operatively coupled a common actuation mechanism (e.g., a capstan) housed (contained) within the drive housing 208 (FIG. 2), and actuation of a drive input associated with the common actuation mechanism causes the jaw cable 428 to move. In other embodiments, however, the ends 430a,b of the jaw cable 428 may be operatively coupled to individual (discrete) actuation mechanisms (e.g., two capstans) housed within the drive housing 208. In such embodiments, actuation of corresponding drive inputs associated with each actuation mechanism will cooperatively cause tension or slack in the jaw cable 428.

Coupling the ends 430a,b of the jaw cable 428 to individual actuation mechanisms (e.g., two capstans) may prove advantageous in providing a "pull-pull" system that facilitates a larger amount of closing and opening forces for the jaws 210, 212, as compared to a common actuation mechanism. With a common (single) actuation mechanism, movement of the actuation mechanism causes equal and opposite movement of the ends 430a,b of the jaw cable 428. With individual actuation mechanisms operating each end 430a,b, however, movement of the two ends 430a,b may be equal and opposite but may also be different since an operator has the ability to ease one end as the other end is pulled. If the movement is different there will be different tensions in each end 430a,b. This may prove advantageous in minimizing load and subsequent wear on the jaw cable 428, thus extending device mission life and enhancing control of closing and opening the jaws 210, 212. Moreover, individual actuation mechanisms operating each end of the jaw cable 428 enables the two longitudinal lengths of the jaw cable 428 to be routed through the joint 206 in asymmetric configurations, which allows load balancing.

Another advantage to coupling the ends 430a,b of the jaw cable 428 to individual actuation mechanisms may be in minimizing cable stretch. For example, if the jaws 210, 212 are closed, the second end 430b will assume a high tensile load and will stretch. In contrast, the first end 430a may be paid out simultaneously to prevent slack, but the tension in the first end 430a can be substantially lower, which reduces the potential for stretch. The importance of this arrangement is that the length of a stretched cable is not as easy to predict as the length of a non-stretched cable, and prediction of cable movement is critical to the control of jaw movement. When an actuation mechanism is commanded to rotate to a specific position (as measured by rotary encoder on the motor) the accuracy of predicting jaw position is influenced by the stretch of the associated cable. By using separate actuation mechanisms, the lower tension (lower stretch) side of the cable loop can be measured to provide a more accurate prediction of jaw position.

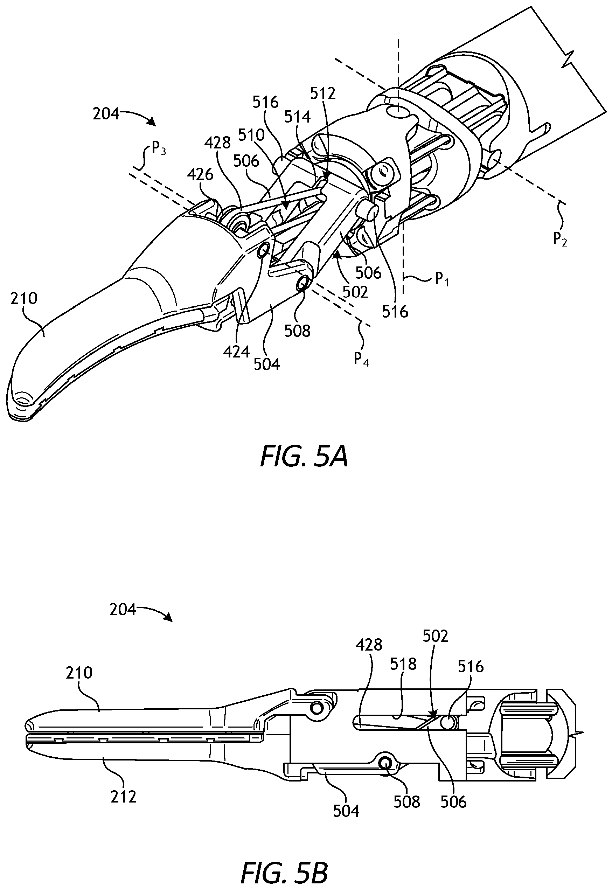

FIGS. 5A and 5B are isometric and side views, respectively, of the end effector 204 with the jaws 210, 212 closed, according to one or more embodiments. The lower jaw 212 is omitted in FIG. 5A to enable viewing of the internal components of the end effector 204. As illustrated, the end effector 204 includes a pivot link 502 operatively coupled to the upper jaw 210. More specifically, the upper jaw 210 provides or otherwise defines opposing legs 504 (one shown, one occluded) that are pivotably coupled to opposing legs 506 of the pivot link 502 at a pivot axle 508.

Referring first to FIG. 5A, a fourth pivot axis P₄ extends through the pivot axle 508 and may be generally perpendicular (orthogonal) to the first pivot axis P₁ and parallel to the second and third pivot axes P₂, P₃. The central pulley 426 is rotatably supported on the jaw axle 424, and the jaw cable 428 loops around the central pulley 426 and includes opposing longitudinal lengths (i.e., ends 430a,b of FIG. 4) that extend proximally through a gap 510 defined between the opposing legs 506 of the pivot link 502.

The jaw cable 428 is operatively coupled to the pivot link 502 such that movement (i.e., longitudinal translation) of the jaw cable 428 correspondingly moves the pivot link 502 in the same direction. For example, a cable anchor 512 may be secured to or otherwise form part of one proximally extending length of the jaw cable 428 and may operatively couple the jaw cable 428 to the pivot link 502. In the illustrated embodiment, the cable anchor 512 comprises a ball crimp receivable within a socket 514 defined by the pivot link 502. In other embodiments, however, the cable anchor 512 may alternatively include, but is not limited to, a weld, an adhesive attachment, a press fit engagement, or any combination of the foregoing. Actuation of the jaw cable 428 such that the cable anchor 512 moves proximally correspondingly causes the pivot link 502 to move in the same direction. Similarly, actuation of the jaw cable 428 such that the cable anchor 512 moves distally correspondingly causes the pivot link 502 to move in the same direction.

Referring to both FIGS. 5A-5B, the pivot link 502 may further provide or otherwise define one or more translation members 516 slidably engageable with the lower jaw 212. More specifically, in some embodiments, the translation members 516 may comprise lateral extensions extending from the pivot link 502 and configured to be received within corresponding jaw slots 518 defined in the lower jaw 212. In some embodiments, as illustrated, the jaw slots 518 may be substantially straight and extend parallel to the longitudinal axis A₂ (FIG. 2) of the end effector 204. In other embodiments, however, the jaw slots 518 may be curved or angled, without departing from the scope of the disclosure.

Receiving the translation members 516 within the corresponding jaw slots 518 effectively constrains one end of the pivot link 502 to the plane defined by the jaw slots 518. Consequently, selectively actuating the jaw cable 428 will correspondingly move the pivot link 502 proximally or distally, and the translation members 516 will be constrained within the jaw slots 518 during such movement. Constraining the translation members 516 within the jaw slots 518 causes the pivot link 502 to act on the upper jaw 210 at the pivot axle 508 similar to a two-bar linkage mechanism, and thereby moves the upper jaw 210 between the open and closed positions.

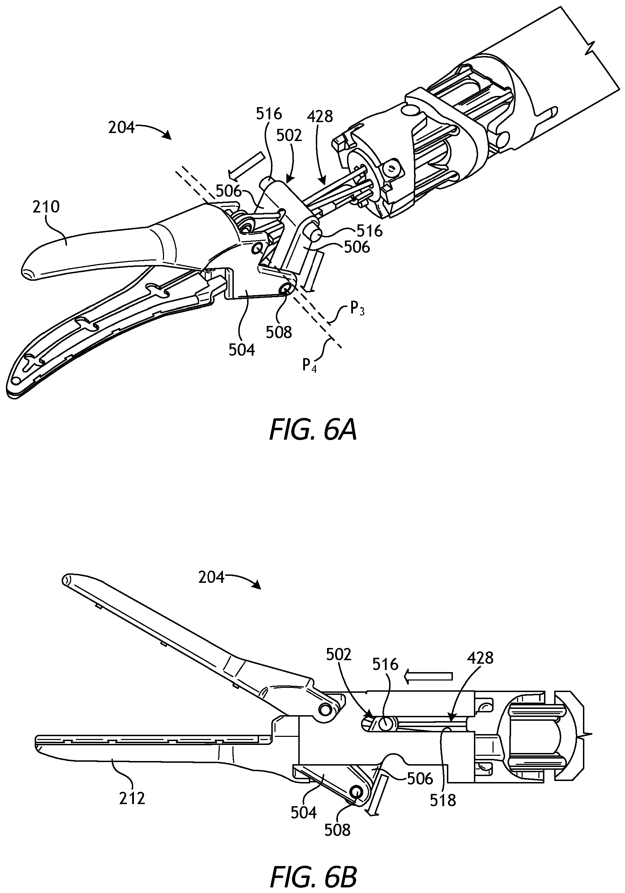

FIGS. 6A and 6B are isometric and side views, respectively, of the end effector 204 with the jaws 210, 212 open, according to one or more embodiments. To move the jaws 210, 212 to the open position, the jaw cable 428 may be actuated to move the pivot link 502 distally. As the pivot link 502 moves distally, the translation members 516 may be constrained within the jaw slots 518 (FIG. 6B), which causes the legs 506 of the pivot link 502 to act on the legs 504 of the upper jaw 210 at the pivot axle 508. More specifically, distal movement of the pivot link 502 forces the legs 504 downward in rotation about the fourth pivot axis P₄ (FIG. 6A), and downward movement of the legs 504 correspondingly causes the upper jaw 210 to pivot about the third pivot axis P₃ to move the upper jaw 210 to the open position. In some embodiments, and because the opposing ends 430a,b (FIG. 4) of the jaw cable 428 may be actuated using individual (discrete) actuation mechanisms, the opening force of the upper jaw 210 may be large enough to use the end effector 204 as a spread dissector.

To move the jaws 210, 212 to the closed position, the jaw cable 428 may be actuated to move the pivot link 502 proximally. The translation members 516 are again constrained within the jaw slots 518 (FIG. 6B) during proximal movement, which causes the legs 506 of the pivot link 502 to pull upward on the legs 504 in rotation about the fourth pivot axis P₄ (FIG. 6A). Upward movement of the legs 504 correspondingly causes the upper jaw 210 to pivot about the third pivot axis P₃ and move the upper jaw 210 back to the closed position.

Therefore, the disclosed systems and methods are well adapted to attain the ends and advantages mentioned as well as those that are inherent therein. The particular embodiments disclosed above are illustrative only, as the teachings of the present disclosure may be modified and practiced in different but equivalent manners apparent to those skilled in the art having the benefit of the teachings herein. Furthermore, no limitations are intended to the details of construction or design herein shown, other than as described in the claims below. It is therefore evident that the particular illustrative embodiments disclosed above may be altered, combined, or modified and all such variations are considered within the scope of the present disclosure. The systems and methods illustratively disclosed herein may suitably be practiced in the absence of any element that is not specifically disclosed herein and/or any optional element disclosed herein. While compositions and methods are described in terms of "comprising," "containing," or "including" various components or steps, the compositions and methods can also "consist essentially of" or "consist of" the various components and steps. All numbers and ranges disclosed above may vary by some amount. Whenever a numerical range with a lower limit and an upper limit is disclosed, any number and any included range falling within the range is specifically disclosed. In particular, every range of values (of the form, "from about a to about b," or, equivalently, "from approximately a to b," or, equivalently, "from approximately a-b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an," as used in the claims, are defined herein to mean one or more than one of the elements that it introduces.

As used herein, the phrase "at least one of" preceding a series of items, with the terms "and" or "or" to separate any of the items, modifies the list as a whole, rather than each member of the list (i.e., each item). The phrase "at least one of" allows a meaning that includes at least one of any one of the items, and/or at least one of any combination of the items, and/or at least one of each of the items. By way of example, the phrases "at least one of A, B, and C" or "at least one of A, B, or C" each refer to only A, only B, or only C; any combination of A, B, and C; and/or at least one of each of A, B, and C.

## Claims

1. An end effector (204), comprising:
a first jaw (210) rotatably coupled to a second jaw (212) at a jaw axle (P3), wherein a single one of the first and second jaws is configured to pivot to articulate the end effector between open and closed positions;
a central pulley (426) rotatably mounted to the jaw axle;
a pivot link (502) rotatably coupled to the first jaw at a pivot axle (508); and
a jaw cable (428) looped around the central pulley and operatively coupled to the pivot link such that linear movement of the jaw cable correspondingly causes the first jaw to rotate relative to the second jaw on the jaw axle and between the open and closed positions.

2. The end effector of claim 1, wherein the first jaw provides one or more legs (506) rotatably coupled to the pivot link at the pivot axle.

3. The end effector of claim 1, further comprising a cable anchor (512) secured to the jaw cable to operatively couple the jaw cable to the pivot link.

4. The end effector of claim 1, wherein the pivot link provides one or more translation members (516) slidably engageable with a corresponding one or more jaw slots defined in the second jaw.

5. The end effector of claim 1, further comprising an electrical conductor (416) terminating at an electrode (418) that supplies electrical energy to at least one of the first and second jaws.

6. A surgical tool (200), comprising:
a drive housing (208);
an elongate shaft (202) that extends from the drive housing;
the end effector (204) of claim 1, wherein the end effector is arranged at a distal end of the elongate shaft;
wherein the jaw cable extends from the drive housing.

7. The surgical tool of claim 6, further comprising a wrist (206) that interposes the end effector and the elongate shaft, wherein the wrist includes:
a first linkage (402a) coupled to the second jaw;
a second linkage (402b) rotatably coupled to the first linkage at a first axle (404a); and
a third linkage (402c) rotatably coupled to the second linkage at a second axle (404b), wherein the second linkage is operatively coupled to the elongate shaft and the jaw cable passes through the first, second, and third linkages.

8. The surgical tool of claim 7, wherein the first and second axles are angularly offset from each other to enable articulation of the wrist in two dissimilar planes.

9. The surgical tool of claim 7, further comprising one or more drive cables (408a, 408b, 408c, 408d) extending from the drive housing and passing through the first, second, and third linkages, wherein selective actuation of the one or more drive cables causes articulation of the wrist.

10. The end effector of claim 9, wherein a distal end of each drive cable terminates at the first linkage or the second jaw.

11. The surgical tool of claim 6, wherein the first jaw provides one or more legs (506) rotatably coupled to the pivot link at the pivot axle.

12. The surgical tool of claim 6, further comprising a cable anchor (512) secured to the jaw cable to operatively couple the jaw cable to the pivot link.

13. The surgical tool of claim 6, wherein the pivot link provides one or more translation members (516) slidably engageable with a corresponding one or more jaw slots defined in the second jaw.

14. The surgical tool of claim 6, further comprising an electrical conductor (416) extending from the drive housing and passing through the first, second, and third linkages, wherein the electrical conductor terminates at an electrode (418) that supplies electrical energy to at least one of the first and second jaws.

## Patentansprüche

1. Endeffektor (204), umfassend:
eine erste Backe (210), die mit einer zweiten Backe (212) an einer Backenachse (P3) drehbar gekoppelt ist, wobei eine einzelne der ersten und der zweiten Backe konfiguriert ist, um zu schwenken, um den Endeffektor zwischen einer geöffneten und einer geschlossenen Position zu bewegen;
eine zentrale Riemenscheibe (426), die an der Backenachse drehbar montiert ist;
ein Schwenkglied (502), das mit der ersten Backe an einer Schwenkachse (508) drehbar gekoppelt ist; und
ein Backenkabel (428), das um die zentrale Riemenscheibe geschlungen und mit dem Schwenkglied derart wirkgekoppelt ist, dass eine lineare Verschiebung des Backenkabels entsprechend bewirkt, dass sich die erste Backe relativ zu der zweiten Backe auf der Backenachse und zwischen der geöffneten und der geschlossenen Position dreht.

2. Endeffektor nach Anspruch 1, wobei die erste Backe ein oder mehrere Beine (506) bereitstellt, die mit dem Schwenkglied an der Schwenkachse drehbar gekoppelt sind.

3. Endeffektor nach Anspruch 1, ferner umfassend einen Kabelanker (512), der an dem Backenkabel gesichert ist, um das Backenkabel mit dem Schwenkglied wirkzukoppeln.

4. Endeffektor nach Anspruch 1, wobei das Schwenkglied ein oder mehrere Translationselemente (516) bereitstellt, die mit einem entsprechenden einen oder mehreren Backenschlitzen gleitbar in Eingriff gebracht werden können, die in der zweiten Backe definiert sind.

5. Endeffektor nach Anspruch 1, ferner umfassend einen elektrischen Leiter (416), der an einer Elektrode (418) endet, die mindestens einer der ersten und der zweiten Backe elektrische Energie zuführt.

6. Chirurgisches Instrument (200), umfassend:
ein Antriebsgehäuse (208);
einen länglichen Schaft (202), der sich von dem Antriebsgehäuse erstreckt;
den Endeffektor (204) nach Anspruch 1, wobei der Endeffektor an einem distalen Ende des länglichen Schafts angeordnet ist;
wobei sich das Backenkabel aus dem Antriebsgehäuse erstreckt.

7. Chirurgisches Instrument nach Anspruch 6, ferner umfassend ein Gelenk (206), das den Endeffektor und den länglichen Schaft zwischen sich einschließt, wobei das Gelenk einschließt:
ein erstes Gestänge (402a), das mit der zweiten Backe gekoppelt ist;
ein zweites Gestänge (402b), das mit dem ersten Gestänge an einer ersten Achse (404a) drehbar gekoppelt ist; und
ein drittes Gestänge (402c), das mit dem zweiten Gestänge an einer zweiten Achse (404b) drehbar gekoppelt ist, wobei das zweite Gestänge mit dem länglichen Schaft wirkgekoppelt ist und das Backenkabel durch das erste, das zweite und das dritte Gestänge verläuft.

8. Chirurgisches Instrument nach Anspruch 7, wobei die erste und die zweite Achse winkelmäßig voneinander versetzt sind, um eine Bewegung des Gelenks in zwei unterschiedlichen Ebenen zu ermöglichen.

9. Chirurgisches Instrument nach Anspruch 7, ferner umfassend ein oder mehrere Antriebskabel (408a, 408b, 408c, 408d), die sich von dem Antriebsgehäuse erstrecken und durch das erste, das zweite und das dritte Gestänge verlaufen, wobei die selektive Bewegung des einen oder der mehreren Antriebskabel eine Bewegung des Gelenks bewirkt.

10. Endeffektor nach Anspruch 9, wobei ein distales Ende jedes Antriebskabels an dem ersten Gestänge oder der zweiten Backe endet.

11. Chirurgisches Instrument nach Anspruch 6, wobei die erste Backe ein oder mehrere Beine (506) aufweist, die mit dem Schwenkglied an der Schwenkachse drehbar gekoppelt sind.

12. Chirurgisches Instrument nach Anspruch 6, ferner umfassend einen Kabelanker (512), der an dem Backenkabel gesichert ist, um das Backenkabel mit dem Schwenkglied wirkzukoppeln.

13. Chirurgisches Instrument nach Anspruch 6, wobei das Schwenkglied ein oder mehrere Translationselemente (516) bereitstellt, die mit einem entsprechenden einen oder mehreren Backenschlitzen gleitbar in Eingriff gebracht werden können, die in der zweiten Backe definiert sind.

14. Chirurgisches Instrument nach Anspruch 6, ferner umfassend einen elektrischen Leiter (416), der sich von dem Antriebsgehäuse erstreckt und durch das erste, das zweite und das dritte Gestänge verläuft, wobei der elektrische Leiter an einer Elektrode (418) endet, die mindestens eine der ersten und der zweiten Backe mit elektrischer Energie versorgt.

## Revendications

1. Effecteur terminal (204), comprenant :
une première mâchoire (210) accouplée de manière rotative à une seconde mâchoire (212) au niveau d'un axe de mâchoire (P3), dans lequel une seule parmi les première et seconde mâchoires est conçue pour pivoter pour articuler l'effecteur terminal entre des positions ouverte et fermée ;
une poulie centrale (426) montée de manière rotative à l'axe de mâchoire ;
une liaison pivot (502) accouplée de manière rotative à la première mâchoire au niveau d'un axe de pivot (508) ; et
un câble de mâchoire (428) enroulé en boucle autour de la poulie centrale et accouplé fonctionnellement à la liaison pivot de telle sorte qu'un mouvement linéaire du câble de mâchoire amène de manière correspondante la première mâchoire à se mettre en rotation par rapport à la seconde mâchoire sur l'axe de mâchoire et entre les positions ouverte et fermée.

2. Effecteur terminal selon la revendication 1, dans lequel la première mâchoire fournit une ou plusieurs pattes (506) accouplées de manière rotative à la liaison pivot au niveau de l'axe de pivot.

3. Effecteur terminal selon la revendication 1, comprenant en outre un ancrage de câble (512) fixé au câble de mâchoire pour accoupler fonctionnellement le câble de mâchoire à la liaison pivot.

4. Effecteur terminal selon la revendication 1, dans lequel la liaison pivot fournit un ou plusieurs éléments de translation (516) pouvant venir en prise de manière coulissante avec une ou plusieurs fentes de mâchoire correspondantes définies dans la seconde mâchoire.

5. Effecteur terminal selon la revendication 1, comprenant en outre un conducteur électrique (416) se terminant au niveau d'une électrode (418) qui alimente en énergie électrique au moins l'une des première et seconde mâchoires.

6. Outil chirurgical (200), comprenant :
un logement d'entraînement (208) ;
une tige allongée (202) qui s'étend à partir du logement d'entraînement ;
l'effecteur terminal (204) selon la revendication 1, dans lequel l'effecteur terminal est agencé au niveau d'une extrémité distale de la tige allongée ;
dans lequel le câble de mâchoire s'étend à partir du logement d'entraînement.

7. Outil chirurgical selon la revendication 6, comprenant en outre une liaison articulée (206) qui s'interpose entre l'effecteur terminal et la tige allongée, dans lequel la liaison articulée comporte :
un premier organe de liaison (402a) accouplé à la seconde mâchoire ;
un deuxième organe de liaison (402b) accouplé de manière rotative au premier organe de liaison au niveau d'un premier axe (404a) ; et
un troisième organe de liaison (402c) accouplé de manière rotative au deuxième organe de liaison au niveau d'un second axe (404b), dans lequel le second organe de liaison est accouplé fonctionnellement à la tige allongée et le câble de mâchoire passe à travers les premier, deuxième et troisième organes de liaison.

8. Outil chirurgical selon la revendication 7, dans lequel les premier et second axes sont décalés angulairement l'un de l'autre pour permettre une articulation de la liaison articulée dans deux plans dissemblables.

9. Outil chirurgical selon la revendication 7, comprenant en outre un ou plusieurs câbles d'entraînement (408a, 408b, 408c, 408d) s'étendant à partir du logement d'entraînement et passant à travers les premier, deuxième et troisième organes de liaison, dans lequel un actionnement sélectif du ou des câbles d'entraînement amène une articulation de la liaison articulée.

10. Effecteur terminal selon la revendication 9, dans lequel une extrémité distale de chaque câble d'entraînement se termine au niveau du premier organe de liaison ou de la seconde mâchoire.

11. Outil chirurgical selon la revendication 6, dans lequel la première mâchoire fournit une ou plusieurs pattes (506) accouplées de manière rotative à la liaison pivot au niveau de l'axe de pivot.

12. Outil chirurgical selon la revendication 6, comprenant en outre un ancrage de câble (512) fixé au câble de mâchoire pour accoupler fonctionnellement le câble de mâchoire à la liaison pivot.

13. Outil chirurgical selon la revendication 6, dans lequel la liaison pivot fournit un ou plusieurs éléments de translation (516) pouvant venir en prise de manière coulissante avec une ou plusieurs fentes de mâchoire correspondantes définies dans la seconde mâchoire.

14. Outil chirurgical selon la revendication 6, comprenant en outre un conducteur électrique (416) s'étendant à partir du logement d'entraînement et passant à travers les premier, deuxième et troisième organes de liaison, dans lequel le conducteur électrique se termine au niveau d'une électrode (418) qui alimente en énergie électrique au moins l'une des première et seconde mâchoires.
